# EUROPÄISCHE PATENTSCHRIFT

(11) **EP 1 476 085 B1**
(45) Veröffentlichungstag und Bekanntmachung des Hinweises auf die Patenterteilung: **03.05.2006**
(21) Anmeldenummer: 03706435.9
(22) Anmeldetag: 06.02.2003
(51) Int. Cl.: A61B 17/15, A61B 17/60

(54) **SCHABLONE ZUR FÜHRUNG EINES CHIRURGISCHEN BEARBEITUNGSWEKZEUGES**
TEMPLATE FOR GUIDING A SURGICAL MACHINING TOOL
GABARIT POUR GUIDER UN OUTIL D'USINAGE CHIRURGICAL

(30) Priorität: 20.02.2002 DE 10207035
(43) Veröffentlichungstag der Anmeldung: 17.11.2004
(73) Patentinhaber: Aesculap AG & Co. KG, 78532 Tuttlingen (DE)
(72) Erfinder: BÖTTIGER, Roland, 78604 Rietheim-Weilheim (DE); FRIEDRICH, Dirk, 78532 Tuttlingen (DE); MARIE, Vincent, 78234 Engen (DE); PFITSCHER, Klaus, 78194 Immendingen (Hattingen) (DE)
(74) Vertreter: Hoeger, Stellrecht & Partner Patentanwälte
(86) Internationale Anmeldenummer: PCT/EP2003/001153
(87) Internationale Veröffentlichungsnummer: WO 2003/070112

(56) Entgegenhaltungen:
- EP-A- 0 555 003
- EP-A- 1 136 041
- US-A- 6 159 217
- US-A1- 2002 133 163
- US-A1- 2002 198 531
- US-B1- 6 267 762

## Beschreibung

Die Erfindung betrifft eine Schablone zur Führung eines chirurgischen gung der Schablone an einem zu bearbeitenden Knochen.

Bel chirurgischen Operationen ist es oft notwendig, einen Knochen zu bearbeiten, beispielsweise müssen beim Ersetzen eines Gelenkes endständige Teile des Knochens entfernt werden, es müssen Anlageflächen für künstliche Gelenkteile bearbeitet werden, und zu diesem Zwecke ist es üblich, die dabei verwendeten Bearbeitungswerkzeuge, beispielsweise oszillierende Sägen oder schnell laufende Fingerfräser, in Schablonen zu führen, die über geeignete Befestigungseinrichtungen an dem zu bearbeitenden Knochen festgelegt werden. Dabei ist es üblich, diese Schablonen direkt am Knochen festzulegen, beispielsweise durch Verschraubung oder durch den Knochen umfangende Bandagen, in all diesen Fällen muß der Knochen in einem Bereich freigelegt werden, der wesentlich größer ist als der eigentlich zu bearbeitende Bereich, um Knochenbereiche freizulegen, die die Schablone halten können. Außerdem ergibt sich bei der Anlage der Schablone am Knochen der Nachteil, daß im unmittelbaren Bearbeitungsbereich die Schablone stören kann.

EP-A-1,136,041 offenbart eine Schablone mit einer Haltzabschnitt, die außerhalb des den Knochen umgebenden Gewebes liegt.

Es ist auch bekannt, derartige Schablonen durch Halterungen am Knochen festzulegen, die in die Markraumhöhle des Knochens eintreten, dies ist aber nur bei speziellen Operationsmöglichkeiten möglich, insbesondere fehlt eine solche Möglichkeit dann, wenn ein Röhrenknochen endständig für die Aufnahme eines künstlichen Gelenkimplantates vorbereitet werden soll.

Es ist Aufgabe der Erfindung, eine gattungsgemäße Schablone so auszubilden, daß sie mit möglichst geringen Eingriffen am Knochen in einer Position festgelegt werden kann, die bei der Bearbeitung des Knochens möglichst wenig stört.

Diese Aufgabe wird bei einer Schablone gemäß Anspruch 1 gelöst.

Die Schablone umfaßt also die drei Knochenschrauben wie Haltepfosten eines Pfahlbaues, diese Knochenschrauben werden in den zu bearbeitenden Knochen eingeschraubt, und dieses Einschrauben kann gegebenenfalls durch kleine Einschnitte im umgebenden Gewebe hindurch erfolgen, ohne daß dieses Gewebe wesentlich geschädigt wird. Die Halteabschnitte liegen dabei außerhalb der Körperoberfläche und im Abstand zu dieser, und an diesen Halteabschnitten wird die Schablone so festgelegt, daß sie ihrerseits im Abstand zur Körperoberfläche gehalten ist. Damit bleibt der eigentlich zu bearbeitende Bereich des Knochens von der Schablone frei und ist für den Operateur frei zugänglich. Dies gilt auch für den Bereich zwischen Schablone und Knochen bzw. Gewebe, bei der Operation kann es für den Operateur durchaus von Bedeutung sein, in diesem Bereich von der Schablone ungestört arbeiten zu können. Der Abstand zwischen Schablone und Knochen muß mindestens so groß sein wie die Höhe des Bearbeitungswerkzeuges, bei minimalinvasiver Operationstechnik muß dieser Abstand mindestens so groß sein wie die Summe aus Werkzeughöhe und umgebender Gewebeschicht. Trotzdem ergibt sich durch die Verwendung von drei derartigen Knochenschrauben eine sehr stabile Festlegung der Schablone am Knochen, so daß die mit Hilfe dieser Schablone ausgeführte Bearbeitung des Knochens sehr präzise erfolgen kann.

Günstig ist es, wenn mindestens eine Knochenschraube eine sogenannte Schanz'sche Schraube ist. Dabei können alle drei Knochenschrauben in dieser Weise ausgebildet sein.

Bei einer bevorzugten Ausführungsform ist dagegen vorgesehen, daß mindestens eine Knochenschraube ein Referenzelement eines Navigationssystemes trägt. Es wird dadurch möglich, eine derartige Knochenschraube mit einer Doppelfunktion zu belegen. Normalerweise werden derartige Referenzelemente eines Navigationssystemes ohnehin in den Knochen eingeschraubt, so daß auf diese Weise möglich ist, mit nur zwei zusätzlichen Knochenschrauben eine sichere Festlegung der Schablone am Knochen in der gewünschten Position zu ermöglichen. Diese Referenzelemente können herkömmliche Reflektoren oder Sender von elektromagnetischer Strahlung oder Ultraschallstrahlung sein.

Besonders vorteilhaft ist es, wenn die Festlegung der Schablone an den Knochenschrauben lösbar ist, so daß durch Lösen der Festlegung die Schablone relativ zu den Knochenschrauben veränderbar ist. Der Operateur hat daher die Möglichkeit, die Schablone in der gewünschten Relativposition zum Knochen festzulegen.

Günstig ist es, wenn zur Festlegung der Schablone an einer Knochenschraube ein Klemmelement vorgesehen ist.

Bei einer ersten bevorzugten Ausführungsform ist vorgesehen, daß die Schablone an zwei Knochenschrauben um die Verbindungsachse der Festlegungspunkte an diesen verdrehbar gelagert ist. Der Operateur kann damit die Schablone um diese Verbindungsachse verschwenken und an der dritten Knochenschraube in der gewünschten Winkelstellung festlegen.

Um dies zu erleichtern, ist es vorteilhaft, wenn an der Schablone im Abstand zu der Verbindungsachse ein Stützelement in Richtung auf die Knochenoberfläche verstellbar gelagert ist, welches bei Anlage an der Knochenfläche beim Verstellen die Schablone relativ zur Verbindungsachse verschwenkt. Das Stützelement wird also an den Knochen angelegt, es kann sich dabei beispielsweise um eine Gewindespindel handeln, die in einem Gewinde der Schablone geführt ist. Durch Verstellen dieser Schablone läßt sich der Schwenkwinkel der Schablone um die Verbindungsachse der Festlegungspunkte verändern, und bei Erreichen der gewünschten Winkelstellung kann diese an der dritten Knochenschraube fixiert werden.

Bei einer besonders bevorzugten Ausführungsform ist vorgesehen, daß die Schablone einen Träger umfaßt, der an den Knochenschrauben festlegbar ist, und daß die übrigen Teile der Schablone an dem Träger gehalten sind. Vorzugsweise ist der Träger als Plattform ausgebildet.

Die übrigen Teile der Schablone können relativ zu dem Träger verschiebbar und in verschiedenen Positionen an diesem festlegbar sein. Insbesondere kann an dem Träger eine Längsführung angeordnet sein, auf der die übrigen Teile der Schablone längsverschieblich gelagert sind. Damit erhält der Operateur eine weitere Verstellmöglichkeit für die Schablone, um diese an die von ihm gewünschte Positionierung anzupassen.

Die übrigen Teile der Schablone können beispielsweise an der Längsführung durch ein Klemmglied festlegbar sein.

Es ist weiterhin vorteilhaft, wenn die Längsführung um eine senkrecht zu ihrer Führungsrichtung verlaufende Schwenkachse verschwenkbar an dem Träger gelagert und in unterschiedlichen Winkellagen an diesem festlegbar ist. Auch die Längsführung kann am Träger durch ein Klemmglied festlegbar sein.

Bei einer weiteren bevorzugten Ausführungsform ist vorgesehen, daß sie einen Führungskörper aufweist, an dem eine Halterung für eine Werkzeugaufnahme längs einer Führung verschieblich gelagert und in unterschiedlichen Positionen festlegbar ist.

Vorteilhaft ist es dabei, wenn längs der Führung ein Mitnehmer verschiebbar ist, der mit der Halterung verbunden ist. Durch Verschieben des Mitnehmers läßt sich somit die Halterung längs der Führung des Führungskörpers verschieben. Die Halterung kann insbesondere quer zur Verschieberichtung der

Führung gegenüber dem Führungskörper verschwenkbar an diesem gelagert und in unterschiedlichen Winkelstellungen festlegbar sein. Damit erhält man für die Halterung eine Positionierungsmöglichkeit gegenüber der Führung sowohl in Längsrichtung der Führung als auch hinsichtlich der Winkelstellung.

Eine besonders günstige Ausgestaltung ergibt sich, wenn die Halterung an dem Mitnehmer verschwenkbar gelagert ist.

Zur Feststellung der Halterung in unterschiedlichen Winkelstellungen können Stellschrauben vorgesehen sein.

An einer Halterung der Schablone kann bei einer weiteren bevorzugten Ausführungsform eine Werkzeugaufnahme lösbar festlegbar sein. Diese Werkzeugaufnahme dient der eigentlichen Führung des Bearbeitungswerkzeuges. Dabei können mehrere unterschiedliche Werkzeugauf nahmen vorhanden sein, die auswechselbar an der Halterung festlegbar sind. Der Operateur hat also die Möglichkeit, für verschiedene Bearbeitungsgänge mit ein und derselben Halterung zu arbeiten und an dieser unterschiedliche Werkzeugaufnahmen festzulegen, die unterschiedliche Bearbeitungsvorgänge ermöglichen.

Die Werkzeugaufnahmen können an der Halterung in unterschiedlichen Positionen festlegbar sein. Beispielsweise kann die Werkzeugaufnahme an der Halterung längs einer Führung verschiebbar und in einer Rasteinrichtung in Positionen mit vorbestimmtem Abstand festlegbar sein.

Insgesamt erhält der Operateur auch dadurch die Möglichkeit, die Lage der Bearbeitungswerkzeuge relativ zum Knochen in der gewünschten Weise einzustellen.

Bei einer bevorzugten Ausführungsform ist vorgesehen, daß die Halterung einen Querarm und an dessen Enden quer von diesem abstehende Haltearme aufweist, an denen die Werkzeugaufnahme gehalten ist. Insbesondere kann die Werkzeugaufnahme dabei zumindest teilweise im Zwischenraum zwischen den Haltearmen angeordnet sein. Dies ergibt eine besonders günstige Anordnung, wobei der gesamte Zwischenraum zwischen den Haltearmen frei bleibt für die Bewegung des Bearbeitungswerkzeuges, der Operateur behält also den freien Zugang zu dem zu bearbeitenden Gebiet des Knochens und wird durch die Halterung der Schablone nicht bei der Bearbeitung gestört.

Bei einer abgewandelten Ausführungsform kann auch vorgesehen sein, daß die Schablone einen Führungskörper aufweist, an dem eine Werkzeugaufnahme unverstellbar festgelegt ist.

Die Variationsmöglichkeit der Verstellung ist besonders groß, wenn der Führungskörper gemäß einer weiteren bevorzugten Ausführungsform am Träger längsverschieblich und auswechselbar gelagert ist. Man erhält also einen modularen Aufbau der Schablone, durch Verwendung unterschiedlicher Führungskörper kann der Operateur unter Beibehaltung des am Knochen festgelegten Trägers ganz unterschiedliche Werkzeugaufnahmen in unterschiedlichen Positionen verwenden, um die jeweiligen Bearbeitungsvorgänge durchführen zu können.

Besonders vorteilhaft ist eine Ausführung, bei der vorgesehen ist, daß an der Schablone eine Aufnahme für ein Referenzelement eines Navigationssystemes angeordnet ist. Insbesondere ist die Aufnahme starr mit einem Teil der Schablone verbunden, an welchem auch die Werkzeugaufnahme gehalten ist. Damit wird es möglich, die Positionierung der Werkzeugaufnahme über das Navigationssystem festzustellen. Wenn ein entsprechendes Referenzelement auch am Knochen angeordnet ist, gibt das Navigationssystem damit Auskunft über die exakte Positionierung der Werkzeugaufnahme relativ zum Knochen.

Die Schablone weist eine Werkzeugaufnahme mit einem Führungsschlitz auf, in welches ein Bearbeitungswerkzeug für einen Knochen einführbar ist. Insbesondere kann die Werkzeugaufnahme auch mehrere unterschiedlich orientierte Führungsschlitze aufweisen.

Die Schablone wird durch die drei Knochenschrauben im Abstand zum Knochen und zur Körperoberfläche sicher gehalten. Eine besonders gute Fixierung erhält man, wenn gemäß der Erfindung zwei Knochenschrauben im wesentlichen in einer Ebene verlaufen und die dritte im Abstand von dieser Ebene zwischen den beiden Knochenschrauben angeordnet ist. Diese Ebene der beiden Knochenschrauben verläuft dabei vorzugsweise quer zur Längsrichtung eines zu bearbeitenden Knochens, während die dritte Knochenschraube dann etwa in der Mitte des Knochens in diesen eingeschraubt ist.

Insbesondere können die beiden Knochenschrauben schräg aufeinander zulaufend orientiert sein, beispielsweise mit einem Winkel zwischen 50° und 120°, in Extremfällen kann dieser Winkel auch noch größer sein, im Extremfall kann er bis zu 180° betragen, in diesen Fällen ist dann die Schablone mit entsprechenden Verlängerungen mit den Knochenschrauben verbunden. Dies ermöglicht dem Operateur eine sehr großflächige Halterung der Schablone am Knochen, d. h. die Halteabschnitte der Knochenschrauben sind auf diese Weise weit voneinander entfernt und bieten eine große Grundfläche für die Befestigung der Schablone. Günstig ist es, wenn der Abstand zwischen dem Schraubabschnitt und dem Halteabschnitt einer Knochenschraube zwischen 10 mm und 150 mm liegt, so daß sichergestellt ist, daß auch bei unterschiedlich ausgebildeter Dicke der den Knochen umgebenden Gewebeschicht in jedem Fall die Schablone im Abstand zur Außenfläche des Körpers angeordnet wird. Dieser Abstand zwischen Schraubabschnitt und Halteabschnitt hängt sehr stark davon ab, wie stark geneigt die Knochenschraube in den Knochen eingeschraubt wird, letztlich wird dieser Abstand dadurch bestimmt, daß zwischen der Schablone einerseits und dem Knochen andererseits ein gewisser Mindestabstand eingehalten werden muß, damit das Werkzeug den Knochen bearbeiten kann und damit gegebenenfalls umgebendes Gewebe zwischen Schablone und Knochen verbleiben kann.

Die nachfolgende Beschreibung bevorzugter Ausführungsformen der Erfindung dient im Zusammenhang mit der Zeichnung der näheren Erläuterung. Es zeigen:
- Figur 1:: einen mit drei Knochenschrauben an einem Knochen festgelegten Träger einer Schablone und einen von diesem entfernten, auf diesen aufschiebbaren Führungskörper mit einer Halterung für eine Werkzeugaufnahme;
- Figur 2:: eine Draufsicht auf die Anordnung der Figur 1 mit auf den Träger aufgeschobenem Führungskörper und in die Halterung eingesetzter Werkzeugaufnahme;
- Figur 3;: eine Vorderansicht der Anordnung der Figur 2;
- Figur 4:: eine Seitenansicht der Anordnung der Figur 2;
- Figur 5:: eine Schnittansicht längs Linie 5-5 in Figur 2;
- Figur 6:: eine Schnittansicht längs Linie 6-6 in Figur 2;
- Figur 7:: eine perspektivische Ansicht eines abgewandelten Ausführungsbeispiels eine Führungskörpers mit einer Werkzeugaufnahme;
- Figur 8:: eine perspektivische Ansicht einer Schablone zur Bestimmung der Knochengröße und zur Orientierung von in den Knochen einzuschraubenden Knochenschrauben und
- Figur 9:: eine schematische Seitenansicht eines Knochens mit einer Schablone, deren dritte Knochenschraube gleichzeitig ein Referenzelement eines Navigationssystemes trägt.

Die Erfindung wird am Beispiel einer Schablone 1 erörtert, die am Ende eines Knochens 2, beispielsweise eines Femurknochens, angeordnet wird, um dort eine Entfernung des Gelenkkopfes und die Ausbildung von Aufnahmeflächen für einen künstlichen Gelenkersatz zu ermöglichen. Dabei kann es sich um verschiedenartige Röhrenknochen handeln, die Anwendung ist nicht auf den Femurknochen beschränkt.

Die Schablone 1 umfaßt einen plattenförmigen Träger 3, von dem auf gegenüberliegenden Seiten zwei koaxial zueinander angeordnete Haltestäbe 4, 5 abstehen, diese sind gegenüber dem Träger 3 um ihre Längsachse verdrehbar im Träger 3 gelagert. Es wäre aber auch möglich, die Haltestäbe 4, 5 drehfest am Träger 3 zu befestigen. Auf dem Träger 3 ist eine Führungsplatte 6 angeordnet, die einen trapezförmigen Querschnitt aufweist und somit den Führungskörper einer Schwalbenschwanzführung ausbildet. Die Führungsplatte 6 ist im Bereich der Haltestäbe 4, 5 um eine senkrecht zum Träger 3 und zur Führungsplatte 6 sowie senkrecht zu den Haltestäben 4 und 5 verlaufende Drehachse verschwenkbar am Träger 3 gelagert, diese Lagerung wird durch einen eine Lageröffnung der Führungsplatte 6 durchsetzenden und am Träger 3 festgelegten Lagerbolzen 7 bewirkt, der in der Zeichnung nur schematisch dargestellt ist. Im Abstand zu dem Lagerbolzen 7 ist in der Führungsplatte 6 ein konzentrisch zu der Drehachse verlaufender, bogenförmiger Schlitz 8 angeordnet, durch den ein am Träger 3 befestigter Gewindebolzen 9 hindurchtritt. Auf diesen ist ein Haltearm 10 aufgesteckt, der durch eine auf den Gewindebolzen 9 aufgeschraubte Flügelmutter 11 gegen die Führungsplatte 6 gespannt werden kann. Dadurch wird einerseits die Führungsplatte 6 in ihrer Winkelstellung um die durch den Lagerbolzen 7 gebildete Schwenkachse festgelegt und andererseits der Haltearm 10 gegenüber einer Drehung relativ zum Gewindebolzen 9.

Bei einem abgewandelten Ausführungsbeispiel kann auch vorgesehen sein, daß der Haltearm 10 am Gewindebolzen 9 gegen Drehung gesichert ist. Dies läßt sich beispielsweise dadurch erreichen, daß der Gewindebolzen 9 einseitig abgeflacht ist und daß eine den Gewindebolzen 9 aufnehmende Bohrung im Haltearm 10 komplementär zum Querschnitt des Gewindebolzens ausgebildet ist. Bei einem solchen Ausführungsbeispiel ist also die Orientierung des Haltearmes 10 relativ zu dem Gewindebolzen 9 und damit relativ zum Träger 3 unveränderlich. An dem Haltearm 10 sind zwei parallel zueinander angeordnete, von diesem quer zur Längsrichtung des Gewindebolzens 9 abstehende Haltestifte 12, 13 befestigt.

Der Gewindebolzen 9 weist eine durchgehende zentrale Bohrung 14 mit einem Innengewinde 15 auf, in dieses Innengewinde 15 ist eine Stellschraube 16 mit einem Drehknopf 17 eingeschraubt, die so tief in den Gewindebolzen 9 einschraubbar ist, daß ihr freies Ende 18, das vorzugsweise zugespitzt ausgebildet ist, durch eine mit der Bohrung 14 fluchtende Durchbrechung 19 des Trägers 3 nach unten hervorsteht. Das freie Ende 18 kann sich dabei am Knochen 2 abstützen, so daß bei Verdrehen der Stellschraube 16 der Träger 3 um eine Drehachse verschwenkt wird, die durch die beiden Haltestäbe 4 und 5 definiert wird.

Die bisher beschriebene Anordnung wird am Knochen 2 durch drei Knochenschrauben 20, 21, 22 gehalten. Es handelt sich dabei um sogenannte Schanz'sche Schrauben, das sind kreiszylindrische Stifte, die an einem Ende einen Gewindeabschnitt 23 tragen und am gegenüberliegenden Ende einen gewindefreien zylindrischen Abschnitt, der nachstehend als Halteabschnitt 24 bezeichnet wird. Zwei Knochenschrauben 20 und 21 werden in einer etwa quer zur Längsrichtung des Knochens 2 verlaufenden Ebene so in den Knochen 2 eingeschraubt, daß sie unter einem Winkel von 50° bis 120° aufeinander zulaufen, d. h. ihre Halteabschnitte 24 divergieren in dieser Ebene. Dieser Winkel kann aber auch noch größer sein und im Grenzfall bis 180° betragen, in einem solchen Falle wird der Träger 3 an den Knochenschrauben über geeignete Zwischenstücke gehalten.

Die dritte Knochenschraube 22 wird im Abstand von der von den beiden Knochenschrauben 20 und 21 aufgespannten Ebene etwa in der Mitte zwischen den beiden Knochenschrauben 20 und 21 etwa senkrecht zur Längsrichtung des Knochens 2 in diesen eingeschraubt. Alle Knochenschrauben 20, 21 und 22 sind so lang, daß die Halteabschnitte 24 außerhalb des Körpers und im Abstand von der Körperoberfläche liegen, und an diesen Halteabschnitten 24 sind Klemmelemente 25, 26, 27 angeordnet, die jeweils einen Haltestab 4, 5 bzw. einen der Lagerstifte 12, 13 aufnehmen. Jedes Klemmelement 25, 26, 27 ist mit einer Klemmschraube 28 versehen, durch die die Klemmelemente 25, 26, 27 so gespannt werden können, daß sie sowohl an den Knochenschrauben 20, 21, 22 als auch an den Haltestäben 4, 5 bzw. den Lagerstiften 12 oder 13 festgeklemmt sind und diese relativ zueinander festlegen.

Diese Klemmelemente können sehr unterschiedlich ausgebildet sein, im dargestellten Ausführungsbeispiel weist jedes Klemmelement zwei Klemmplatten 29, 30 auf, die durch die Klemmschraube 28 gegeneinander geklemmt werden. In jeder Klemmplatte 29, 30 ist in einer Bohrung 31, 32 jeweils eine Knochenschraube bzw. ein Haltestab oder ein Lagerstift aufgenommen, und beim Spannen werden die Knochen schrauben, Haltestäbe und Lagerstifte gegen die jeweils andere Klemmplatte gespannt und dadurch festgelegt.

Es ist auf diese Weise möglich, den Träger 3 stabil am Knochen 2 anzuordnen, und zwar in einem solchen Abstand von diesem, daß der Träger 3 außerhalb des den Knochen umgebenden Gewebes und im Abstand zur Körperoberfläche angeordnet wird, die Winkelstellung relativ zu der von den Haltestäben 4 und 5 ausgebildeten Schwenkachse kann mit Hilfe der Stellschraube 16 innerhalb gewisser Grenzen eingestellt und dann durch die Klemmelemente 25, 26, 27 fixiert werden. Die Führungsplatte 6 wird relativ zum Träger 3 in unterschiedlichen Winkelstellungen durch die Flügelmutter 11 fixiert, dies gilt ebenso für den Haltearm 10.

Der Operateur hat also allein durch das Setzen der drei Knochenschrauben 20, 21 und 22 und durch das Festspannen der Knochenschrauben 20, 21, 22 und der Flügelmutter 11 die Möglichkeit, den Träger 3 mit der Führungsplatte 6 in der gewünschten Höhe und mit der gewünschten Verschwenkung des Trägers 3 und mit der gewünschten Winkelstellung der Führungsplatte 6 zu fixieren.

Der Träger 3 mit der Führungsplatte 6 bildet eine Basis zur Positionierung der übrigen Teile der Schablone 1. Dabei können diese übrigen Teile modulartig und auswechselbar ausgebildet sein, so daß je nach Bedarf auf diese Basis unterschiedliche Module aufgesetzt werden können, an denen jeweils Führungselemente für Bearbeitungswerkzeuge in unterschiedlicher Weise angeordnet sind.

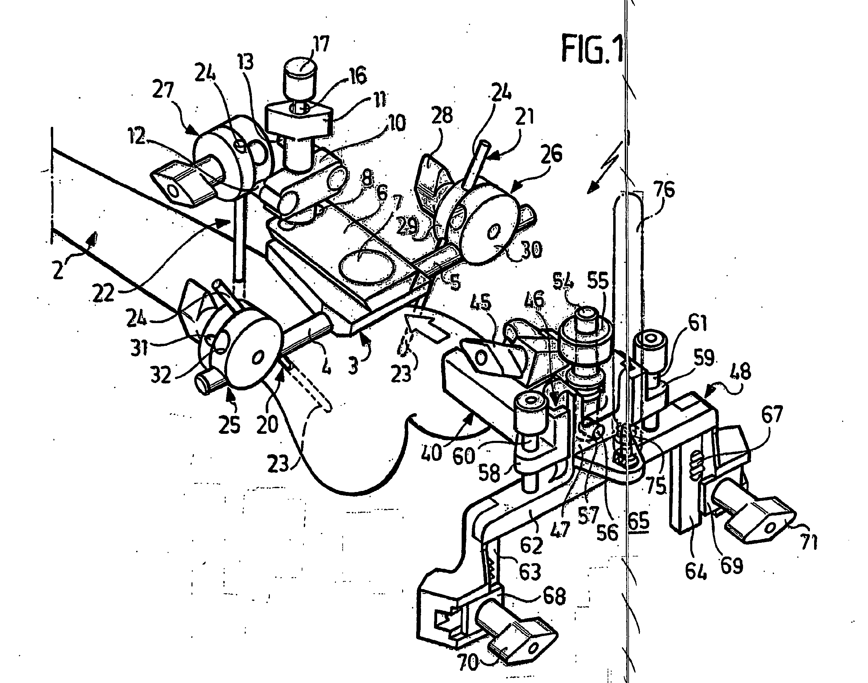

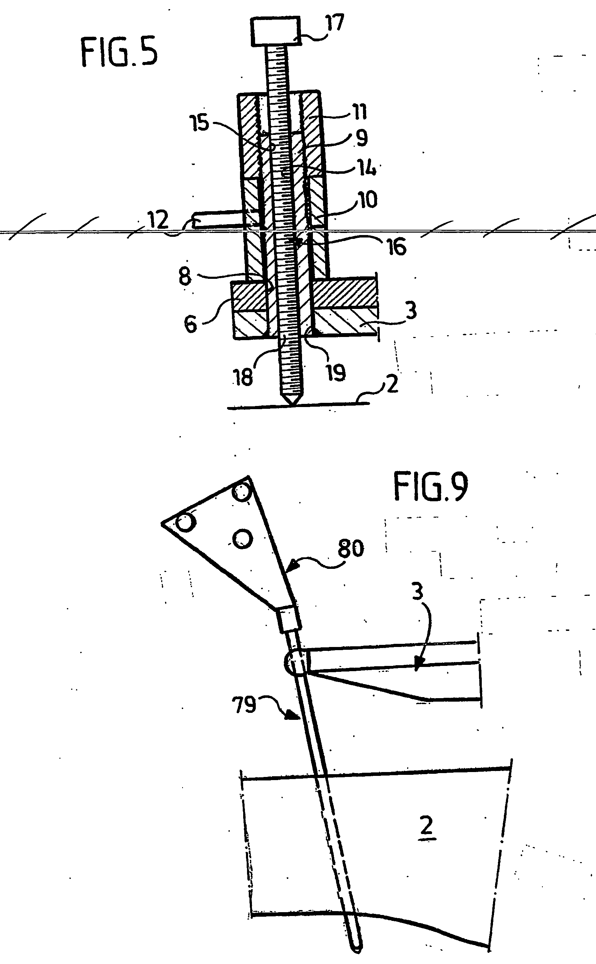

Bei einem ersten in den Figuren 1 bis 6 dargestellten Ausführungsbeispiel umfassen diese übrigen Teile der Schablone 1 einen Führungskörper 40, der im wesentlichen plattenförmig aufgebaut ist und an seiner Unterseite eine Längsnut 41 aufweist, deren Querschnitt komplementär zum Querschnitt der trapezförmigen Führungsplatte 6 ist. Der Führungskörper 40 kann in Längsrichtung so über die Führungsplatte 6 geschoben werden, daß diese in die Längsnut 41 eintaucht, es entsteht dadurch eine Längsführung nach Art einer Schwalbenschwanzführung (Figur 6). An dem Führungskörper 40 ist in einer schräg verlaufenden Bohrung 42 eine Spannschraube 43 drehbar gelagert, die an einem Ende eine an der Seitenfläche der Führungsplatte 6 anlegbare Spannplatte 44 trägt und auf die auf dem anderen Ende eine Spannmutter 45 aufschraubbar ist, durch die die Spannplatte 44 gegen die Seitenfläche der Führungsplatte 6 spannbar ist. Dadurch kann der Führungskörper 40 in Längsrichtung der Führungsplatte 6 relativ zu dieser festgeklemmt werden (Figur 6).

Im aufgeschobenen Zustand steht der Führungskörper 40 einseitig über den Träger 3 vor, und zwar auf der der dritten Knochenschraube 22 gegenüberliegenden Seite. An diesem Ende trägt der Führungskörper 40 eine senkrechte Führung 46 mit einer senkrechten, im Querschnitt T-förmigen Nut, die zu der der Knochenschraube 22 abgewandten Seite des Führungskörpers 40 hin offen ist. Diese Nut verläuft senkrecht zu der Längsnut 41 im Führungskörper 40.

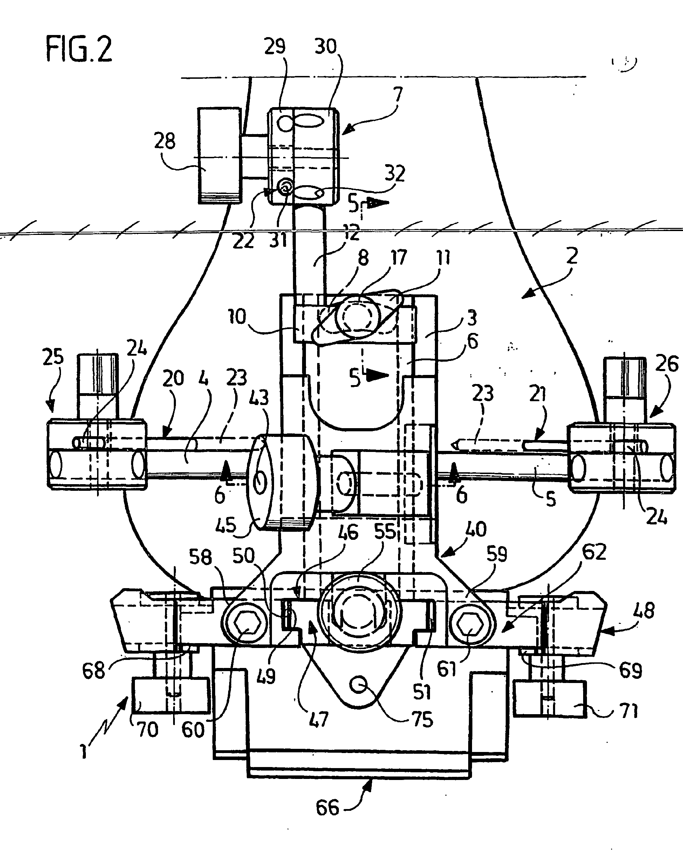

In dieser Nut ist eine im Querschnitt T-förmige Führungsleiste 47 einer Halterung 48 längsverschieblich geführt, diese Führungsleiste 47 ist etwas schmaler ausgebildet als die nutförmige Führung 46, so daß zwischen den Seitenkanten 50 der Füihrungsleiste 47 und den Seitenwänden 49 der Führung 46 ein Zwischenraum 51 verbleibt (Figur 2).

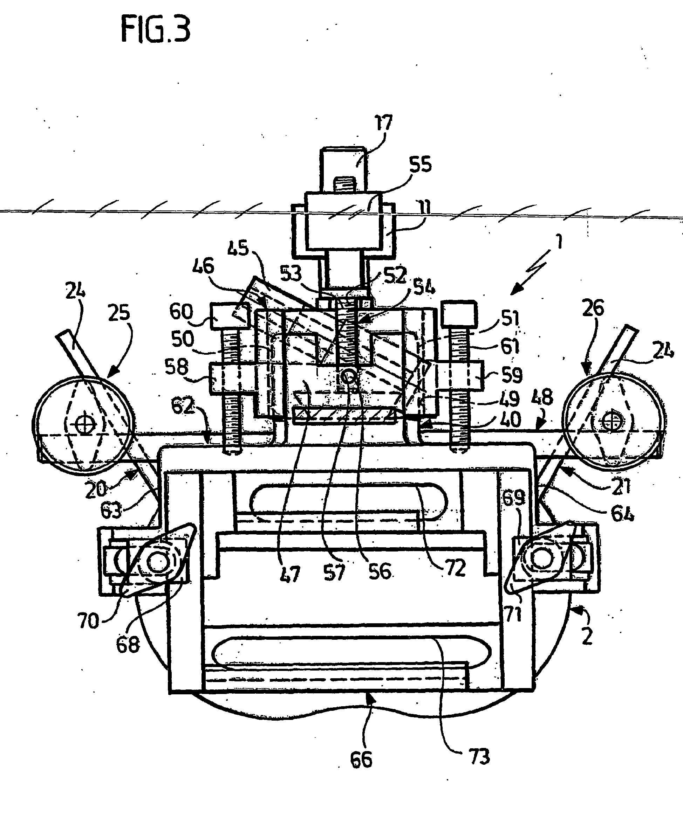

In einer parallel zu der Führung 46 verlaufenden Halbbohrung 52 des Führungskörpers 40 ist längsverschieblich ein mit einem Außengewinde 53 versehener Gelenkbolzen 54 aufgenommen, auf den eine sich am Rand der Halbbohrung 52 abstützende Stellmutter 55 aufgeschraubt ist. Der Gelenkbolzen 54 trägt in seinem unteren, aus der Halbbohrung 52 austretenden Bereich senkrecht von ihm abstehend einen kreiszylindrischen Lagerstift 56, der in eine Lageröffnung 57 der Halterung 48 eingreift und dadurch eine Schwenklagerung für die Halterung 48 ausbildet. Die Achse dieser Schwenklagerung verläuft in der Mitte der nutförmigen Führung 46 senkrecht zu deren Längsausdehnung und ermöglicht der Führungsleiste 47 eine geringfügige Verschwenkung innerhalb der nutförmigen Führung 46, die Verschwenkbewegung wird dadurch begrenzt, daß bei einer Verschwenkung von beispielsweise maximal ± 8° aus der Senkrechten heraus die Seitenkante 50 der Führungsleiste 47 an der Seitenwand 49 der Führung 46 anschlägt. Die geringfügige Verschwenkbarkeit wird also durch den Zwischenraum 51 ermöglicht. Zur Fixierung des Schwenkwinkels der Halterung 48 sind zu beiden Seiten der Führung 46 des Führungskörpers 40 Gewindeösen 58, 59 angeformt, in die jeweils eine Stellschraube 60 bzw. 61 eingeschraubt ist (Figur 3). Diese Stellschrauben 60 und 61 stützen sich an einem mit der Führungsleiste 47 starr verbundenen, senkrecht zu dieser verlaufenden Querarm 62 der Halterung 48 ab und bilden so Anschläge aus, die eine Verschwenkung des Querarmes 62 und damit der gesamten Halterung 48 begrenzen. Zur Auswahl der entsprechenden Winkelstellung werden diese Stellschrauben 60, 61 zunächst ein wenig herausgedreht, so daß die Halterung 48 in der nutförmigen Führung 46 frei versehwenkbar ist, sobald die gewünschte Winkelstellung erreicht ist, werden beide Stellschrauben 60, 61 bis zur Anlage an dem Querarm 62 eingeschraubt, und damit ist die Winkelstellung in beiden Richtungen fixiert.

Der Gelenkbolzen 54 ist durch den Eingriff des Lagerstiftes 56 in die Lageröffnungen 57 gegen eine Drehung um die Längsachse des Gelenkbolzens 54 gesichert, verdreht man daher die Stellmutter 55 auf dem Gelenkbolzen 54, so wird der Gelenkbolzen 54 mehr oder weniger aus der Halbbohrung 52 herausgezogen und hebt dabei in entsprechender Weise die Halterung 48 gegenüber dem Führungskörper 40 an. In aus der Zeichnung nicht ersichtlicher Weise kann vorgesehen werden, daß die Stellmutter 55 sich nicht nur in einer Richtung am Führungskörper 40 abstützt, sondern in beiden entgegengesetzten Richtungen, das kann beispielsweise durch den Eingriff eines Vorsprunges am Führungskörper 40 in eine Ringnut an der Stellmutter 55 erreicht werden.

An den beiden Enden des Querarmes 62 sind senkrecht von diesem abstehende, parallel zueinander verlaufende Haltearme 63 und 64 angeordnet, die zwischen sich einen Zwischenraum 65 ausbilden, in den ein Führungsblock 66 einschiebbar ist. Die Haltearme 63 und 64 bilden dabei Führungsschienen für den Führungsblock 66 aus. An diesen Haltearmen 63 und 64 sind in Verschieberichtung des Führungsblockes 66 hintereinander mehrere zahnförmige Vorsprünge 67 angeordnet, die mit entsprechenden, in der Zeichnung nicht dargestellten Vorsprüngen am Führungsblock 66 zusammenwirken und somit längs der Haltearme 63 und 64 ein gerastertes Vorschieben des Führungsblockes 66 ermögsprünge 67 an den Haltearmen zwischen die Vorsprünge an dem Führungsblock 66 eingreifen und dadurch eine unverschiebbare, stufenförmig gerasterte Festlegung des Führungsblockes 66 an den Haltearmen 63, 64 ermöglicht. Die Klemmplatten 68, 69 werden durch In die Haltearme 63, 64 einschraubbare Klemmschrauben 70, 71 gegen den Führungsblock 66 und die Haltearme 63, 64 gespannt.

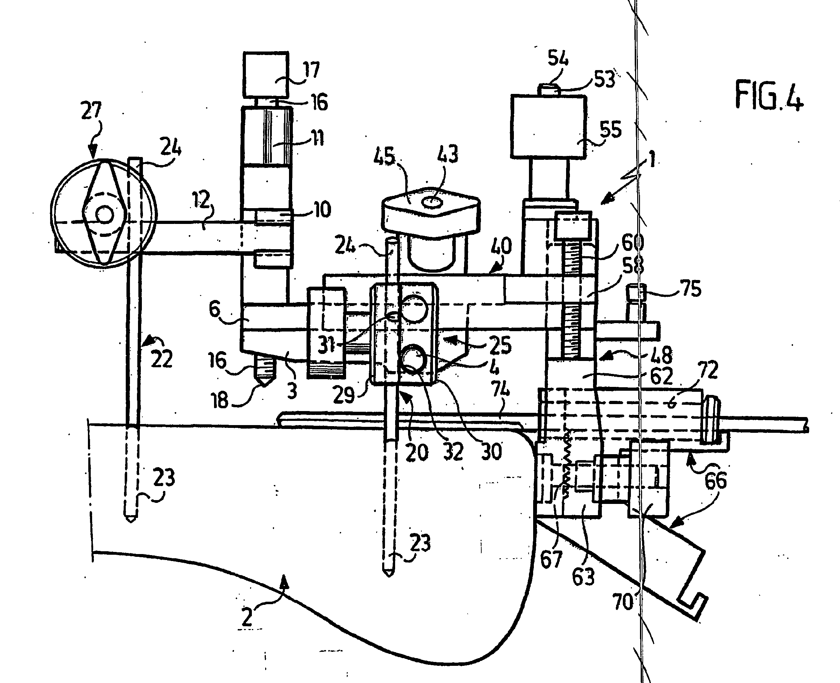

In den Zwischenraum 65 sind unterschiedlich geformte Führungsblöcke 66 einsetzbar. Im dargestellten Ausführungsbeispiel ist ein Führungsblock 66 verwendet, der zwei geradlinige Führungsschlitze 72, 73 aufweist. Ein Führungsschlitz verläuft in einer Ebene, die senkrecht zur Führungsleiste 47 angeordnet ist, ein zweiter Führungsschlitz 73 ist gegenüber dieser Ebene geneigt und in Richtung auf den Knochen schräg ansteigend ausgebildet (Figur 4). Diese Führungsschlitze 72 und 73 sind relativ zum Knochen 1 durch die beschriebenen Einstellmöglichkeiten so zu positionieren, daß ein durch einen Führungsschlitz 72 hindurchgeschobenes Bearbeitungswerkzeug 74 in ihnen in der durch den Führungsschlitz 72 aufgespannten Ebene geführt wird und in dieser Ebene den Knochen 2 bearbeiten kann. Das Bearbeitungswerkzeug 74 kann beispielsweise ein schnell laufender Fingerfräser mit sehr kleinem Durchmesser sein oder ein Sägeblatt einer oszillierenden Säge.

Mit dem in den Figuren 1 bis 6 dargestellten Führungsblock 66 können auf diese Weise am Knochen 2 zwei gegeneinander geneigte ebene Anlageflächen erzeugt werden, bei entsprechend geänderter Anordnung von einem oder mehreren Führungsschlitzen in einem Führungsblock sind auch entsprechend andere Flächen zu bearbeiten. Zu diesem Zweck stehen unterschiedliche Führungsblöcke mit unterschiedlicher

Anordnung der Führungsschlitze zur Verfügung, die je nach Bedarf an der Halterung ausgewechselt werden können. Durch die gerasterte Festlegung der Führungsblöcke 66 an der Halterung 48 kann auch unterschiedlichen Abmessungen des Knochens Rechnung getragen werden.

Im übrigen ist durch die vielfältigen Verstellmöglichkeiten sichergestellt, daß auch die Positionierung und Orientierung der bearbeiteten Flächen am Knochen in der gewünschten Weise vorgenommen werden kann.

Um dies zu erleichtern, ist an der Halterung 48 ein Haltevorsprung 75 angeordnet, auf den ein Referenzelement 76 eines Navigationssystemes aufgesetzt werden kann. In der Darstellung der Figur 1 ist dieses Referenzelement 76 schematisch durch ein strichpunktiertes Rohr symbolisiert, hier kann es sich um an sich bekanntere Referenzelemente handeln, wie sie bei Navigationssystemen eingesetzt werden, also beispielsweise um Reflektoren oder aktive Sender von elektromagnetischer Strahlung oder Ultraschallwellen. Dieses Referenzelement 76 zeigt über das Navigationssystem die Positionierung der Halterung 48 im Raum an, und bei Vergleich mit einem weiteren Referenzelement, das beispielsweise über eine Knochenschraube direkt am Knochen 2 gehalten ist, läßt sich somit auch die Relativposition der Halterung 48 gegenüber dem Knochen 2 jederzeit genau bestimmen. Der Operateur kann daher unter Verwendung der vorstehend beschriebenen Einstellmöglichkeiten die Führungsschlitze des Führungsblocks 66 für alle zu bearbeitenden Flächen so positionieren, daß eine exakte Positionierung und Orientierung der bearbeiteten Knochenflächen sichergestellt wird. Referenzelemente für das Navigationssystem können auch direkt an den Führungsblocks 66 befestigt werden, so daß auch deren Lage im Raum unmittelbar feststellbar ist.

Dabei wird der Operateur so vorgehen, daß er zunächst die Lage des Trägers 3 und die Orientierung der Führungsplatte 6 festlegt, erst dann werden die Einstellungen am Führungskörper 40 vorgenommen.

Bei der Bearbeitung des Knochens 2 kann der Führungskörper 40 von dem in dieser Weise festgelegten Träger 3 abgenommen und durch einen anderen Führungskörper ersetzt werden, der gegebenenfalls verschieden aufgebaut ist und Führungsschlitze unterschiedlicher Ausgestaltung trägt.

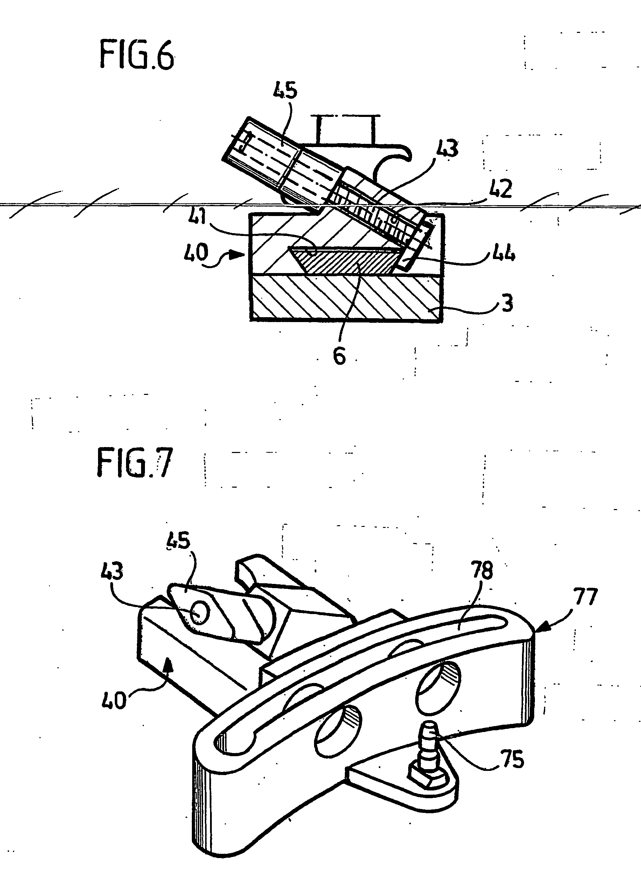

Ein Beispiel eines solchen abgeänderten Führungskörpers ist in Figur 7 dargestellt.

Dieser ist zunächst ähnlich ausgebildet wie der Führungskörper 40, entsprechende Teile tragen daher dieselben Bezugszeichen. Im Unterschied zu dem Führungskörper 40 der Figuren 1 bis 6 fehlt jedoch bei dem Führungskörper der Figur 7 eine Führung, die der Führung 46 entspricht. Statt dessen ist mit dem Führungskörper 40 starr ein Führungsblock 77 verbunden, der seinerseits einen Führungsschlitz 78 aufweist. Dieser erstreckt sich in einer Ebene, die senkrecht auf der Längsnut 41 steht.

Der Führungskörper 40 der Figur 7 kann in gleicher Weise auf den Träger 3 aufgeschoben werden wie der Führungskörper 40 beim Ausführungsbeispiel der Figuren 1 bis 6, beim Einführen eines Bearbeitungswerkzeuges in den Führungsschlitz 78 wird aber nicht eine Fläche an der Oberseite des Knochens 2 bearbeitet, sondern eine Fläche an der Stirnseite des Knochens 2.

Prinzipiell können auch noch mehr derartige Führungskörper 40 mit unterschiedlichen Führungsschlitzen vorgesehen sein, die dann dem Operateur wahlweise zur Verfügung stehen.

Bei den bisher beschriebenen Ausführungsbeispielen wird der Träger 3 über drei im wesentlichen gleich ausgebildeten Knochenschrauben 20, 21, 22 am Knochen 2 gehalten. Grundsätzlich ist es möglich, eine der Knochenschrauben durch eine Knochenschraube 79 zu ersetzen, die ein Referenzelement 80 eines Navigationssystems trägt. Derartige Knochenschrauben werden bei Operationen dieser Art in jedem Falle benötigt, um die Position des Knochens in einem Koordinatensystem des Navigationssystems zu erfassen. Es ist nun möglich, eine Knochenschraube dadurch einzusparen, daß einer der Knochenschrauben eine Doppelfunktion zugewiesen wird, nämlich einmal eine Tragefunktion für den Träger 3 und zum anderen eine Tragefunktion für das Referenzelement 80. Dies ist in dem Ausführungsbeispiel der Figur 9 schematisch dargestellt.

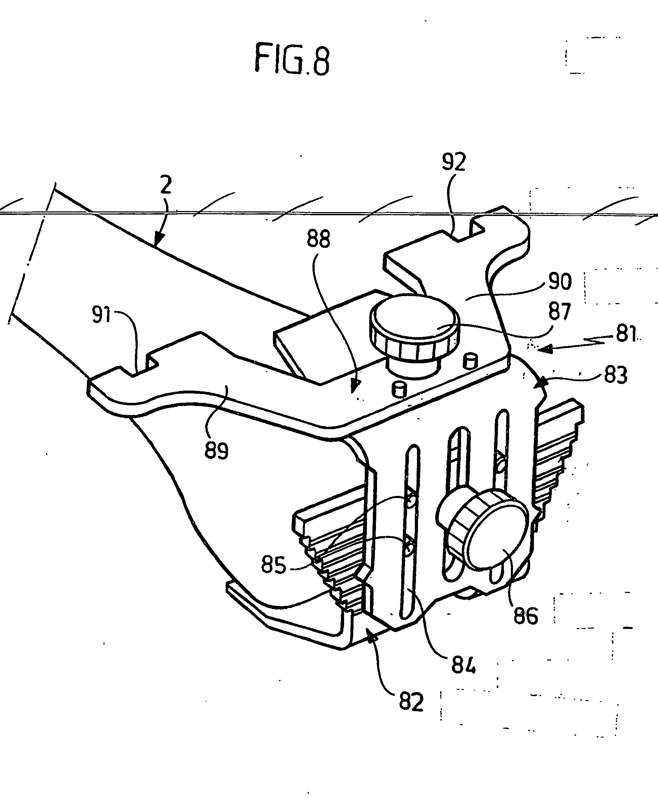

Die Anordnung des Trägers 3 in einem Abstand vom Knochen 2 ermöglicht es, im Zwischenraum zwischen Träger 3 und Knochen 2 Bearbeitungsschritte vorzunehmen. Um sicherzustellen, daß diese Bearbeitungsschritte auch nicht durch die in den Knochen 2 eingeschraubten Knochenschrauben 20 und 21 behindert werden, kann mittels einer Schablone 81 die Anordnung dieser Knochenschrauben 20 und 21 so gesteuert werden, daß der Arbeitsbereich von den Knochenschrauben 20 und 21 freigehalten wird. Eine solche Schablone 81 ist in Figur 8 dargestellt. Sie umfaßt zwei L-förmige Hälften 82, 83, die längs einer Führung 84, 85 relativ zueinander verschieblich und durch eine Spannschraube 86 gegeneinander spannbar sind. Diese Hälften 82 und 83 können an die Oberseite und die Unterseite eines Knochens 2 angelegt werden und dienen zunächst für den Operateur als Meßvorrichtung zur Bestimmung der Größe des Knochens. Gleichzeitig kann an der Oberseite dieser Schablone 81 in genau definierter Position mittels einer Spannschraube 87 ein Bügel 88 mit seitlich abstehenden Armen 89, 90 angeklemmt werden, der an jedem Arm 89, 90 eine seitlich offene Ausnehmung 91 bzw. 92 aufweist. Wenn der Operateur beim Einschrauben der Knochenschrauben 20 und 21 diese durch die Ausnehmungen 91, 92 hindurchführt, ist sichergestellt, daß die Knochenschrauben 20 und 21 divergierend in den Knochen eingesetzt sind, da der Abstand der Ausnehmungen 91, 92 voneinander wesentlich größer ist als der Durchmesser des Knochens 2. Die Ausnehmungen 91 und 92 bilden dabei eine Einschraublehre für die Knochenschrauben 20 und 21. Der horizontale Abstand der Ausnehmungen 91 und 92 muß dabei größer sein als der mögliche Verschiebeweg des Werkzeuges in einem Führungsschlitz. Die Dicke des Materials der Hälfte 83 definiert dabei den Mindestabstand des Trägers 3 vom Knochen.

## Patentansprüche

1. Schablone (1) zur Führung eines chirurgischen Bearbeitungswerkzeuges (74) mit einer Werkzeugaufnahme (66, 77) mit einem Führungsschlitz (72, 73, 78), in welchen das Bearbeitungswerkzeug (74) für den Knochen (2) einführbar ist, und mit einer Befestigungseinrichtung zur Festlegung der Schablone (1) an dem zu bearbeitenden Knochen (2), welche Befestigungseinrichtung genau drei Knochenschrauben (20, 21, 22; 79) mit jeweils einem in den Knochen (2) einschraubbaren Schraubabschnitt (23) und einem im Abstand dazu angeordneten Halteabschnitt (24) umfaßt, wobei der Abstand zwischen Schraubabschnitt (23) und Halteabschnitt (24) so groß ist, daß der Halteabschnitt (24) bei in den Knochen (2) eingeschraubter Knochenschraube (20, 21, 22; 79) außerhalb des den Knochen (2) umgebenden Gewebes im Abstand von der Körperoberfläche liegt, wobei weiterhin zwei von den drei Knochenschrauben (20, 21) im wesentlichen in einer Ebene verlaufen und die dritte Knochenschraube (22; 79) im Abstand von dieser Ebene zwischen den beiden Knochenschrauben (20, 21) angeordnet ist, und wobei die Schablone (1) im Bereich der Halteabschnitte (24) mittels genau drei Verbindungselementen (25, 26, 27) an den Knochenschrauben (20, 21, 22; 79) festgelegt ist.

2. Schablone nach Anspruch 1, **dadurch gekennzeichnet, daß** mindestens eine Knochenschraube (20, 21, 22) eine Schanz'sche Schraube ist.

3. Schablone nach Anspruch 1 oder 2, **dadurch gekennzeichnet, daß** mindestens eine Knochenschraube (79) ein Referenzelement (80) eines Navigationssystemes trägt.

4. Schablone nach einem der voranstehenden Ansprüche, **dadurch gekennzeichnet, daß** die Festlegung der Schablone (1) an den Knochenschrauben (20, 21, 22; 79) lösbar ist.

5. Schablone nach Anspruch 4, **dadurch gekennzeichnet, daß** zur Festlegung der Schablone (1) an einer Knochenschraube (20, 21, 22; 79) ein Klemmelement (25, 26, 27) vorgesehen ist.

6. Schablone nach einem der voranstehenden Ansprüche, **dadurch gekennzeichnet, daß** die Schablone (1) an zwei Knochenschrauben (20, 21) um die Verbindungsachse der Festlegungspunkte an diesen verdrehbar gelagert ist.

7. Schablone nach Anspruch 6, **dadurch gekennzeichnet, daß** an der Schablone (1) im Abstand zu der Verbindungsachse ein Stützelement (16, 18) in Richtung auf die Knochenoberfläche verstellbar gelagert ist, welches bei Anlage an der Knochenoberfläche beim Verstellen die Schablone (1) relativ zur Verbindungsachse verschwenkt.

8. Schablone nach einem der voranstehenden Ansprüche, **dadurch gekennzeichnet, daß** sie einen Träger (3) umfaßt, der an den Knochenschrauben (20, 21, 22; 79) festlegbar ist, und daß die übrigen Teile der Schablone (1) an dem Träger (3) gehalten sind.

9. Schablone nach Anspruch 8, **dadurch gekennzeichnet, daß** der Träger (3) als Plattform ausgebildet ist.

10. Schablone nach einem der Ansprüche 8 oder 9, **dadurch gekennzeichnet, daß** die übrigen Teile der Schablone (1) relativ zu dem Träger (3) verschiebbar und in verschiedenen Positionen an diesem festlegbar sind.

11. Schablone nach Anspruch 10, **dadurch gekennzeichnet, daß** an dem Träger (3) eine Längsführung (6) angeordnet ist, auf der die übrigen Teile der Schablone (1) längsverschieblich gelagert sind.

12. Schablone nach Anspruch 11, **dadurch gekennzeichnet, daß** die übrigen Teile der Schablone (1) an der Längsführung (6) durch ein Klemmglied (43, 44, 45) festlegbar sind.

13. Schablone nach einem der Ansprüche 11 oder 12, **dadurch gekennzeichnet, daß** die Längsführung (6) um eine senkrecht zu ihrer Führungsrichtung verlaufende Schwenkachse verschwenkbar an dem Träger (3) gelagert und in verschiedenen Winkellagen an diesem festlegbar ist.

14. Schablone nach Anspruch 13, **dadurch gekennzeichnet, daß** die Längsführung (6) am Träger (3) durch ein Klemmglied (9, 11) festlegbar ist.

15. Schablone nach einem der voranstehenden Ansprüche, **dadurch gekennzeichnet, daß** sie einen Führungskörper (40) aufweist, an dem eine Halterung (48) für eine Werkzeugaufnahme (66) längs einer Führung (46) verschieblich gelagert und in unterschiedlichen Positionen festlegbar ist.

16. Schablone nach Anspruch 15, **dadurch gekennzeichnet, daß** längs der Führung (46) ein Mitnehmer (54, 56) verschiebbar ist, der mit der Halterung (48) verbunden ist.

17. Schablone nach einem der Ansprüche 15 oder 16, **dadurch gekennzeichnet, daß** die Halterung (48) quer zur Verschieberichtung der Führung (46) gegenüber dem Führungskörper (40) verschwenkbar an diesem gelagert und in unterschiedlichen Winkelstellungen festlegbar ist.

18. Schablone nach den Ansprüchen 16 und 17, **dadurch gekennzeichnet, daß** die Halterung (48) an dem Mitnehmer (54, 56) verschwenkbar gelagert ist.

19. Schablone nach Anspruch 18 oder 19, **dadurch gekennzeichnet, daß** zur Feststellung der Halterung in unterschliedlichen Winkel stellungen Stellschrauben (60, 61) vorgesehen sind.

20. Schablone nach einem der voranstehenden Ansprüche, **dadurch gekennzeichnet, daß** sie eine Halterung (48) aufweist, an welcher eine Werkzeugaufnahme (66) lösbar festlegbar ist.

21. Schablone nach Anspruch 20, **dadurch gekennzeichnet, daß** mehrere unterschiedliche Werkzeugaufnahmen (66) vorhanden sind, die auswechselbar an der Halterung (48) festlegbar sind.

22. Schablone nach einem der Ansprüche 20 oder 21, **dadurch gekennzeichnet, daß** die Werkzeugaufnahme (66) an der Halterung (48) in unterschiedlichen Positionen festlegbar ist.

23. Schablone nach Anspruch 22, **dadurch gekennzeichnet, daß** die Werkzeugaufnahme (66) an der Halterung (48) längs einer Führung (63, 64) verschiebbar und durch eine Rasteinrichtung (67) in Positionen mit vorbestimmtem Abstand festlegbar ist.

24. Schablone nach einem der Ansprüche 20 bis 23, **dadurch gekennzeichnet, daß** die Halterung (48) einen Querarm (62) und an dessen Enden quer von diesem abstehende Haltearme (63, 64) aufweist, an denen die Werkzeugaufnahme (66) gehalten ist.

25. Schablone nach Anspruch 24, **dadurch gekennzeichnet, daß** die Werkzeugaufnahme (66) zumindest teilweise im Zwischenraum (65) zwischen den Haltearmen (63, 64) angeordnet ist.

26. Schablone nach einem der Ansprüche 1 bis 14, **dadurch gekennzeichnet, daß** sie einen Führungskörper (40) aufweist, an dem eine Werkzeugaufnahme (77) unverstellbar festgelegt ist.

27. Schablone nach einem der Ansprüche 11 bis 14 und nach einem der Ansprüche 15 bis 26, **dadurch gekennzeichnet, daß** der Führungskörper (40) am Träger (3) längsverschieblich und auswechselbar gelagert ist.

28. Schablone nach einem der voranstehenden Ansprüche, **dadurch gekennzeichnet, daß** an der Schablone (1) eine Aufnahme (75) für ein Referenzelement (76) eines Navigationssystemes angeordnet ist.

29. Schablone nach Anspruch 28, **dadurch gekennzeichnet, daß** die Aufnahme (75) starr mit einem Teil (48) der Schablone (1) verbunden ist, an welchem eine Werkzeugaufnahme (66, 77) gehalten ist.

30. Schablone nach einem der voranstehenden Ansprüche, **dadurch gekennzeichnet, daß** die Werkzeugaufnahme (66) mehrere unterschiedlich orientierte Führungsschlitze (72, 73) aufweist.

31. Schablone nach einem der voranstehenden Ansprüche, **dadurch gekennzeichnet, daß** die beiden Knochenschrauben (20, 21) schräg aufeinander zulaufend orientiert sind.

32. Schablone nach Anspruch 31, **dadurch gekennzeichnet, daß** der Winkel zwischen den beiden Knochenschrauben (21, 22) zwischen 50° und 120° liegt.

33. Schablone nach einem der voranstehenden Ansprüche, **dadurch gekennzeichnet, daß** der Abstand zwischen dem Schraubabschnitt (23) und dem Halteabschnitt (24) einer Knochenschraube (20, 21, 22; 79) zwischen 10 mm und 150 mm liegt.

## Claims

1. A gauge (1) for guiding a surgical machining tool (74), comprising a tool holder (66, 77) with a guide slot (72, 73, 78), into which the machining tool (74) for the bone (2) is insertable, and a fixing means for securing the gauge (1) to the bone (2) to be machined, which fixing means comprises precisely three bone screws (20, 21, 22; 79) each having a threaded portion (23) which can be screwed into the bone (2), and a holding portion (24) spaced apart from the threaded portion (23), wherein the distance between the threaded portion (23) and the holding portion (24) is sufficiently great that, when the bone screw (20, 21, 22; 79) is screwed into the bone (2), the holding portion (24) lies outside the tissue surrounding the bone (2) and is spaced apart from the surface of the body, furthermore wherein two of the three bone screws (20, 21) extend substantially in one plane and the third bone screw (22; 79) is arranged between the two bone screws (20, 21) at a distance from this plane, and wherein the gauge (1) is secured to the bone screws (20, 21, 22; 79) in the region of the holding portions (24) by means of precisely three connecting members (25, 26, 27).

2. A gauge according to claim 1, **characterised in that** at least one bone screw (20, 21, 22) is a Schanz screw.

3. A gauge according to claim 1 or 2, **characterised in that** at least one bone screw (79) carries a reference element (80) of a navigation system.

4. A gauge according to any one of the preceding claims, **characterised in that** the gauge (1) is detachably secured to the bone screws (20, 21, 22; 79).

5. A gauge according to claim 4, **characterised in that** a clamping member (25, 26, 27) is provided for securing the gauge (1) to a bone screw (20, 21, 22; 79).

6. A gauge according to any one of the preceding claims, **characterised in that** the gauge (1) is mounted on two bone screws (20, 21) so as to be rotatable at the securing points about the connecting axis thereof.

7. A gauge according to claim 6, **characterised in that** a supporting member (16, 18) is mounted on the gauge (1), with spacing from the connecting axis, so as to be adjustable in the direction of the surface of the bone and, when resting against the surface of the bone during adjustment, pivots the gauge (1) relative to the connecting axis.

8. A gauge according to any one of the preceding claims, **characterised in that** it comprises a carrier (3) which is securable to the bone screws (20, 21, 22; 79), and **in that** the remaining parts of the gauge (1) are held on the carrier (3).

9. A gauge according to claim 8, **characterised in that** the carrier (3) is formed as a platform.

10. A gauge according to either one of claims 8 and 9, **characterised in that** the remaining parts of the gauge (1) are displaceable relative to the carrier (3) and are securable thereto in different positions.

11. A gauge according to claim 10, **characterised in that** a longitudinal guide (6), on which the remaining parts of the gauge (1) are longitudinally displaceably mounted, is arranged on the carrier (3).

12. A gauge according to claim 11, **characterised in that** the remaining parts of the gauge (1) are securable to the longitudinal guide (6) by a clamping component (43, 44, 45).

13. A gauge according to either one of claims 11 and 12, **characterised in that** the longitudinal guide (6) is mounted on the carrier (3) so as to be pivotable about a pivoting axis extending perpendicularly to its guiding direction and is securable to the carrier (3) in different angular positions.

14. A gauge according to claim 13, **characterised in that** the longitudinal guide (6) is securable to the carrier (3) by a clamping component (9, 11).

15. A gauge according to any one of the preceding claims, **characterised in that** it has a guide body (40) on which a mounting (48) for a tool holder (66) is mounted so as to be displaceable along a guide (46) and is securable in different positions.

16. A gauge according to claim 15, **characterised in that** a driver (54, 56) is displaceable along the guide (46) and is connected to the mounting (48).

17. A gauge according to either one of claims 15 and 16, **characterised in that** the mounting (48) is mounted on the guide body (40) so as to be pivotable relative thereto transversely to the displacement direction of the guide (46) and is securable in different angular positions.

18. A gauge according to claims 16 and 17, **characterised in that** the mounting (48) is pivotably mounted on the driver (54, 56).

19. A gauge according to claim 18 or 19, **characterised in that** adjusting screws (60, 61) are provided for fixing the mounting in different angular positions.

20. A gauge according to any one of the preceding claims, **characterised in that** it has a mounting (48) to which a tool holder (66) is detachably securable.

21. A gauge according to claim 20, **characterised in that** a plurality of different tool holders (66) are available which are interchangeably securable to the mounting (48).

22. A gauge according to either one of claims 20 and 21, **characterised in that** the tool holder (66) is securable to the mounting (48) in different positions.

23. A gauge according to claim 22, **characterised in that** the tool holder (66) is displaceable on the mounting (48) along a guide (63, 64) and is securable by a locking means (67) in positions with predetermined spacing.

24. A gauge according to any one of claims 20 to 23, **characterised in that** the mounting (48) has a transverse arm (62) and holding arms (63, 64) which project transversely from the ends thereof and on which the tool holder (66) is held.

25. A gauge according to claim 24, **characterised in that** the tool holder (66) is at least partly arranged in the gap (65) between the holding arms (63, 64).

26. A gauge according to any one of claims 1 to 14, **characterised in that** it has a guide body (40) to which a tool holder (77) is fixedly secured.

27. A gauge according to any one of claims 11 to 14 and according to any one of claims 15 to 26, **characterised in that** the guide body (40) is mounted on the carrier (3) so as to be exchangeable and longitudinally displaceable.

28. A gauge according to any one of the preceding claims, **characterised in that** a mount (75) for a reference element (76) of a navigation system is arranged on the gauge (1).

29. A gauge according to claim 28, **characterised in that** the mount (75) is rigidly connected to a part (48) of the gauge (1) on which a tool holder (66, 77) is held.

30. A gauge according to any one of the preceding claims, **characterised in that** the tool holder (66) has a plurality of differently orientated guide slots (72, 73).

31. A gauge according to any one of the preceding claims, **characterised in that** the two bone screws (20, 21) are orientated so as to extend obliquely towards one another.

32. A gauge according to claim 31, **characterised in that** the angle between the two bone screws (21, 22) lies between 50° and 120°.

33. A gauge according to any one of the preceding claims, **characterised in that** the distance between the threaded portion (23) and the holding portion (24) of a bone screw (20, 21, 22; 79) lies between 10 mm and 150 mm.

## Revendications

1. Gabarit (1) pour guider un outil d'usinage chirurgical (74), comprenant un porte-outil (66, 77) avec une fente de guidage (72, 73, 78) dans laquelle peut être inséré l'outil d'usinage (74) pour l'os (2), et comprenant également un dispositif de fixation pour fixer et immobiliser le gabarit (1) sur l'os (2) à usiner, ledit dispositif de fixation comportant exactement trois vis à os ou broches (20, 21, 22 ; 79) avec chacune un tronçon de vissage (23) pouvant être vissé dans l'os (2) et un tronçon de support (24) situé à distance du précédent, la distance entre le tronçon de vissage (23) et le tronçon de support (24) étant d'une grandeur telle, que le tronçon de support (24), lorsque la vis à os (20, 21, 22 ; 79) est vissée dans l'os (2), se situe en-dehors des tissus entourant l'os (2), à distance de la surface extérieure du corps, deux (20, 21) des trois vis à os s'étendant par ailleurs sensiblement dans un plan et la troisième vis à os (22 ; 79) étant agencée à distance de ce plan, entre lesdites deux vis à os (20, 21), et le gabarit (1) étant fixé aux vis à os (20, 21, 22 ; 79), dans la zone des tronçons de support (24), au moyen exactement de trois éléments de liaison (25, 26, 27).

2. Gabarit selon la revendication 1, **caractérisé en ce qu'**au moins une vis à os (20, 21, 22) est une vis ou broche dite de Schanz.

3. Gabarit selon la revendication 1 ou 2, **caractérisé en ce qu'**au moins une vis à os (79) porte un élément de référence (80) d'un système de navigation.

4. Gabarit selon l'une des revendications précédentes, **caractérisé en ce que** la fixation du gabarit (1) sur les vis à os (20, 21, 22; 79) est amovible.

5. Gabarit selon la revendication 4, **caractérisé en ce que** pour la fixation du gabarit (1) sur une vis à os (20, 21, 22 ; 79) il est prévu un élément de serrage (25, 26, 27).

6. Gabarit selon l'une des revendications précédentes, **caractérisé en ce que** le gabarit (1) est monté sur deux vis à os (20, 21) de manière à pouvoir tourner autour de l'axe de liaison des points de fixation sur celles-ci.

7. Gabarit selon la revendication 6, **caractérisé en ce que** sur le gabarit (1), à distance de l'axe de liaison, est monté un élément d'appui (16, 18), qui est déplaçable en direction de la surface de l'os, et qui en cas d'appui sur la surface de l'os lors du déplacement, fait pivoter le gabarit (1) par rapport à l'axe de liaison.

8. Gabarit selon l'une des revendications précédentes, **caractérisé en ce qu'**il comprend un élément porteur (3) qui peut être fixé aux vis à os (20, 21, 22 ; 79), et **en ce que** les autres parties du gabarit (1) sont maintenues sur l'élément porteur (3).

9. Gabarit selon la revendication 8, **caractérisé en ce que** l'élément porteur (3) est réalisé en tant que plate-forme.

10. Gabarit selon l'une des revendications 8 ou 9, **caractérisé en ce que** les autres parties ou pièces du gabarit (1) peuvent coulisser par rapport à l'élément porteur (3) et peuvent être fixées ou immobilisées dans différentes positions sur celui-ci.

11. Gabarit selon la revendication 10, **caractérisé en ce que** sur l'élément porteur (3) est aménagée une glissière de guidage longitudinale (6) sur laquelle sont montées longitudinalement coulissantes les autres pièces du gabarit (1).

12. Gabarit selon la revendication 11, **caractérisé en ce que** lesdites autres pièces du gabarit (1) peuvent être fixées ou immobilisées sur la glissière de guidage longitudinale (6), par un organe de serrage (43, 44, 45).

13. Gabarit selon l'une des revendications 11 ou 12, **caractérisé en ce que** la glissière de guidage longitudinale (6) est montée sur l'élément porteur (3) de manière à pouvoir pivoter autour d'un axe de pivotement s'étendant perpendiculairement à sa direction de guidage, et peut être fixée ou immobilisée dans différentes positions angulaires sur ledit élément porteur.

14. Gabarit selon la revendication 13, **caractérisé en ce que** la glissière de guidage longitudinale (6) peut être fixée ou immobilisée sur l'élément porteur (3) au moyen d'un organe de serrage (9, 11).

15. Gabarit selon l'une des revendications précédentes, **caractérisé en ce qu'**il comprend un corps de guidage (40) sur lequel un support (48) pour un porte-outil (66) est monté coulissant le long d'une glissière de guidage (46), et peut être fixé ou immobilisé dans différentes positions.

16. Gabarit selon la revendication 15, **caractérisé en ce que** le long de la glissière de guidage (46) peut coulisser un entraîneur (54, 56) qui est relié au support (48).

17. Gabarit selon l'une des revendications 15 ou 16, **caractérisé en ce que** le support (48) est monté sur le corps de guidage (40) de manière à pouvoir pivoter par rapport à celui-ci, transversalement à la direction de coulissement de la glissière de guidage (46), et peut être fixé ou immobilisé dans différentes positions angulaires.

18. Gabarit selon les revendications 16 et 17, **caractérisé en ce que** le support (48) est monté pivotant sur l'entraîneur (54, 56).

19. Gabarit selon la revendication 18, **caractérisé en ce que** des vis de réglage (60, 61) sont prévues pour fixer ou immobiliser le support dans différentes positions angulaires.

20. Gabarit selon l'une des revendications précédentes, **caractérisé en ce qu'**il comprend un support (48) sur lequel peut être fixé de manière amovible un porte-outil (66).

21. Gabarit selon la revendication 20, **caractérisé en ce que** son prévus plusieurs porte-outils (66) différents, qui peuvent être fixés de manière interchangeable au support (48).

22. Gabarit selon l'une des revendications 20 ou 21, **caractérisé en ce que** le porte-outil (66) peut être fixé ou immobilisé dans différentes positions sur le support (48).

23. Gabarit selon la revendication 22, **caractérisé en ce que** le porte-outil (66) peut coulisser sur le support (48) le long d'une glissière de guidage (63, 64) et peut être fixé ou immobilisé par un dispositif d'encliquetage (67), dans des positions à distance prédéterminée.

24. Gabarit selon l'une des revendications 20 à 23, **caractérisé en ce que** le support (48) présente un bras transversal (62) et aux extrémités de celui-ci, en saillie transversale de celui-ci, des bras de support (63, 64) par lesquels est supporté le porte-outil (66).

25. Gabarit selon la revendication 24, **caractérisé en ce que** le porte-outil (66) est disposé, au moins en partie, dans l'espace intermédiaire (65) entre les bras de support (63, 64).

26. Gabarit selon l'une des revendications 1 à 14, **caractérisé en ce qu'**il comprend un corps de guidage (40) sur lequel est fixé de manière non réglable, un porte-outil (77).

27. Gabarit selon l'une des revendications 11 à 14 et selon l'une des revendications 15 à 26, **caractérisé en ce que** le corps de guidage (40) est monté de manière à pouvoir coulisser longitudinalement et de manière interchangeable sur l'élément porteur (3).

28. Gabarit selon l'une des revendications précédentes, **caractérisé en ce que** sur le gabarit (1) est agencé un élément de montage (75) pour un élément de référence (76) d'un système de navigation.

29. Gabarit selon la revendication 28, **caractérisé en ce que** l'élément de montage (75) est relié de manière rigide à une partie (48) du gabarit (1), sur laquelle est maintenue un porte-outil (66, 77).

30. Gabarit selon l'une des revendications précédentes, **caractérisé en ce que** le porte-outil (66) présente plusieurs fentes de guidage (72, 73) qui sont orientées différemment.

31. Gabarit selon l'une des revendications précédentes, **caractérisé en ce que** les deux vis à os (20, 21) sont orientées de manière à être dirigées l'une vers l'autre en biais.

32. Gabarit selon la revendication 31, **caractérisé en ce que** l'angle entre les deux vis à os (20, 21) se situe entre 50° et 120°.

33. Gabarit selon l'une des revendications précédentes, **caractérisé en ce que** la distance entre le tronçon de vissage (23) et le tronçon de support (24) d'une vis à os (20, 21, 22 ; 79) se situe entre 10 mm et 150 mm.
